(19) Europäisches Patentamt
European Patent Office
Office européen des brevets

(11) Publication number: **0 053 448**
**B1**

(12) **EUROPEAN PATENT SPECIFICATION**

(45) Date of publication of patent specification: **11.09.85**

(51) Int. Cl.⁴: **A 61 K 7/06**

(21) Application number: **81305376.6**

(22) Date of filing: **12.11.81**

(54) **Pre-shampoo type hair treatment composition.**

(30) Priority: **28.11.80 JP 167863/80**

(43) Date of publication of application:
**09.06.82 Bulletin 82/23**

(45) Publication of the grant of the patent:
**11.09.85 Bulletin 85/37**

(84) Designated Contracting States:
**AT BE CH DE FR IT LI NL**

(56) References cited:
**EP-A-0 052 943**
**DE-A-3 106 974**
**US-A-3 958 581**
**US-A-3 961 634**
**US-A-4 080 310**

**CHEMICAL ABSTRACTS, vol. 89, no. 2, 10th July 1978, page 358, no. 11968k, Columbus, Ohio, USA**

**The file contains technical information submitted after the application was filed and not included in this specification**

(73) Proprietor: **KAO SOAP CO., LTD.**
**1-1, Nihonbashi Kayaba-cho, Chuo-ku**
**Tokyo (JP)**

(72) Inventor: **Matsunaga, Kinjiro**
**Ooaza Wado No. 331-21 Miyashiro-machi**
**Minamisaitama-gun Saitama-ken (JP)**
Inventor: **Tsushima, Rikio**
**4-21-302 Akiba-cho**
**Wakayama-shi Wakayama-ken (JP)**
Inventor: **Okumura, Takeo**
**6-22-19 Nakashizu**
**Sakura-shi Chiba-ken (JP)**

(74) Representative: **Evans, David Charles et al**
**F.J. CLEVELAND & COMPANY 40-43, Chancery Lane**
**London, WC2A 1JQ (GB)**

**EP 0 053 448 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Field of the Invention

This invention relates to a pre-shampoo type hair treatment composition and more particularly, to a pre-shampoo type hair treatment comprising derivatives of keratin material and cationic polymer.

Description of the Prior Art:

Hair is very readily stained such as by dirt from outside or decomposition products or oxides of sebum secreted from scalp. For the purpose of removing the stains, it is general to wash the hair with shampoo compositions. The shampoo compositions used in the washing of hair comprise as their principal component surface active agents such as anionic surface active agents, amphoteric surface active agents and the like, so that when hair is washed with such compositions, not only the stains but also the sebum necessary for imparting suppleness to the hair is also washed away. The hair from which sebum has been washed away is poor to the touch and is hard to comb or brush, and is thus very difficult to handle with the attendant disadvantage that the hair is readily damaged causing split-ends and broken hairs. In order to prevent the hair the deterioration accompanied by washing, there are added to shampoo compositions additives such as oils and fats or polymeric compounds to improve the styling texture of the hair which has been washed.

On the other hand, pre-shampoo type hair treatment compositions containing oils such as liquid lanolin have recently been developed and sold and gained public favor as an epoch-making article for protecting hair. That is, when hair prior to washing is treated with a pre-shampoo hair treatment composition and then washed as usual, damage to the hair that would be caused by washing and rinsing and also by finishing or styling of the hair such as drying by dryers can be prevented, thus imparting conditioning effects to the hair.

However, where hair is protectively treated with such hair treatment compositions containing oils such as lanolin as their principal component, they show disadvantages that the styling texture becomes dull and the treated hair often becomes sticky to the touch. Accordingly, there is a demand for a further improvement and study of this treatment.

Collagen hydrolysates are known *per se* as is their use either alone or in conjunction with cationic polymers in hair treatment compositions, see for example US—A—3968 581, USA—4080310, and Chemical Abstracts Vol. 89, Abstract No. 11968K, 1978. Further, it is known from USA—3961 634 that oxidation products of keratin *per se* are useful in hair bleaching compositions including the oxidizing agent hydrogen peroxide, to limit damage to the hair.

According to the invention, there is provided a pre-shampoo hair treatment composition which comprises a suspension or solution in a polar solvent, of at least one keratin derivative selected from 1) salts with bases of products obtained by oxidation of keratin materials, and 2) salts with bases of derivatives obtained by chemically modifying the mercapto group of a product obtained by reduction of keratin material, wherein said derivatives are selected from

$-SCH_2COOH$, $-SCH_2CH_2COOH$, $-SCHCOOH$
| (on CH:) $CH_2COOH$

$-SCHCH_2COOH$ (on CH: $CH_3$), $-SCH_2CHCOOH$ (on CH: $CH_3$),

$-SSO_3H$, $-SCH_2CH_2SO_3H$, 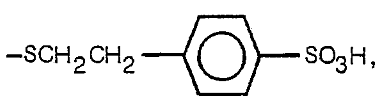,

$-SCH_2CH_2CONHCCH_2SO_3H$ (on C: $CH_3$ above and $CH_3$ below), and $SCH_2CH_2SO_3CH_2COOH$.

The derivative is preferably used in an amount of 0.05 to 5 wt% of the pre-shampoo treatment composition. The pre-shampoo treatment composition further comprises, according to a preferred aspect of the invention, at least one cationic polymer in a predetermined amount.

The derivatives of keratin materials used in the present invention are prepared by oxidising keratin materials and converting the resulting products into salts; with bases and by reducing keratin materials, chemically modifying the resulting mercapto groups and converting the derivatives so obtained to obtain derivatives and then they are converted into salts with bases.

The starting keratin materials include, for example, wool, animal hair, human hair, feathers, claws, horns, hooves and scale, among which wool, human hair and feathers are preferably used. These keratin materials may be subjected to the oxidation or reduction reaction directly but, if necessary, they may be cut or reduced to pieces having a suitable size, or may be subjected to pretreatments such as washing and defatting.

The keratin materials are treated by any of the following methods.

(1) Oxidation Reaction

The oxidation of keratin materials is feasible by any known methods (N. H. Leon; Textile Progress, Vol. 7, page 1 (1975)). Oxidizing agents are preferably organic or inorganic ones of the type which acts electrophilically on the disulfide bonds (S—S bonds) in the keratin structure. Examples of the oxidizing agents include organic peracids, inorganic peroxo acids or their salts, permanganic acid or its salts, chromic acid or related compounds, halogens, peroxides and oxyacids or their salts, among which the organic peracids such as peracetic acid, performic acid and perbenzoic acid are most preferred.

The oxidation reaction is conducted in liquid media using oxidizing agents in excess with respect to the disulfide bonds in the keratin material, ordinarily in amounts of two equivalents or more, preferably 4—10 equivalents of the sulfide bonds. The reaction is feasible under acidic or alkaline conditions and is preferably conducted under acidic conditions and particularly weakly acidic conditions. The reaction temperature and pressures vary depending on the types of the oxidizing agent and keratin material and are not critical. In general, room temperature is sufficient, but, if necessary, heat may be applied. Atmospheric pressure is sufficient but the reaction may be conducted under reduced pressure or under pressure.

By this, the disulfide bonds of keratin material are converted into sulfo groups.

(2) Reduction Reaction and Chemical Modification Reaction

Reducing agents employed for reducing keratin materials are preferably organic or inorganic reducing agents of the type which convert the disulfide bonds in the keratin structure into mercapto groups (—SH) and generally acts nucleophilically on the disulfide bond. Examples of the reducing agents include organic reducing agents such as 2-mercaptoethanol, thioglycollic acid, benzylmercaptan, 1,4-dithiothreitol and tributylphosphine, and inorganic reducing agents such as sodium hydrogensulfite, sulfides such as sodium hydrosulfide, metallic hydrides such as lithium aluminium hydride.

The amount of the reducing agent is usually in the range of 2—10 equivalents of the disulfide bonds in keratin material. The pH of the reaction system is in the range of 2—12, preferably 6—11. Outside the range, undesirable hydrolysis takes place at the same time. Room temperature is sufficient but heat may be applied to shorten the reaction time. The reaction time is ordinarily in the range of 2—3 hours or more. Since it is necessary that the mercapto group produced by the reduction is not substantially oxidized, the reduction operation should conveniently be carried out in an atmosphere of inert gas to give good results.

The mercapto groups of product thus obtained by the reduction of keratin material is then chemically modified to obtain derivatives thereof. The derivatives of the mercapto groups are

$$-SCH_2COOH,\ -SCH_2CH_2COOH,\ -\underset{\displaystyle CH_2COOH}{\underset{|}{SCH}}COOH$$

$$-S\underset{\displaystyle CH_3}{\underset{|}{CH}}CH_2COOH,\ -SCH_2\underset{\displaystyle CH_3}{\underset{|}{CH}}COOH,$$

$-SSO_3H$, $-SCH_2CH_2SO_3H$, 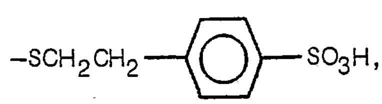

$$-SCH_2CH_2CONH\overset{\displaystyle CH_3}{\overset{|}{\underset{\displaystyle CH_3}{\underset{|}{C}}}}CH_2SO_3H,\qquad SCH_2CH_2SO_3CH_2COOH,$$

among which $-SCH_2COOH$, $-\underset{\displaystyle CH_2COOH}{\underset{|}{SCH}}COOH$ are preferable.

The chemical modification of the mercapto group is known per se and can be conducted, for example, based on procedures known from N. H. Leon; Textile Progress, Vol. 7, page 1 (1975), "Yuki Ioo Kagobutsu (Organic Sulfur Compounds)" written by Shigeru Daikyo and published by Kagaku Dojin (1968) and

"Kobunshi Jikkengaku Koza" written by Masami Oku, Vol. 12, Kyoritsu Shuppan (1957). Typical methods are below.

1 Method utilizing the nucleophilic substitution reaction of SH group

$$K—SH+R—L \rightarrow K—S—R+HL$$

(in which K represents a residue of keratin compound, R represents a chemically modifying group to be introduced, and L represents a leaving atom or group such as a halogen atom or an acid residue). Compounds reacting by this method include, for example, halogen compounds such as iodoacetic acid, bromoacetic acid and chloroacetic acid.

2 Methods utilizing the nucleophilic addition reaction of SH group with a double bond existing between carbon atoms

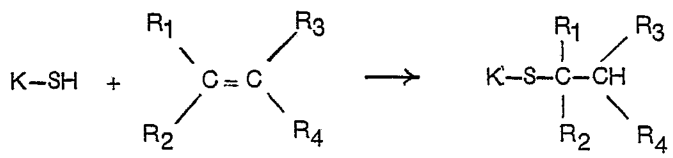

(in which at least one of $R_1$, $R_2$, $R_3$, and $R_4$ represents a carboxyl group or sulfo group and the other represent an alkyl group or hydrogen atom, and K has the same meaning as defined hereinbefore).

Compounds reacting by this method include, for example, acrylic acid, methacrylic acid, crotonic acid, maleic acid, fumaric acid, vinyl, cargoxymethyl sulfone, vinylsulfonic acid, p-styrenesulfonic acid and 2-acrylamido-2-methylpropanesulfonic acid.

3 Method using the substitution reaction between SH group and sulfite compound

$$K—SH+NaHSO_3 \rightarrow K—S—SO_3H$$

$$K—SH+Na_2SO_3 \xrightarrow[\text{air}]{} K—S—SO_3H$$

(in which K has the same meaning as defined above).

The products obtained by the method (1) or (2) should preferably have an average molecular weight of 30,000 — 100,000.

However, the products obtained by the methods (1) and (2) are insoluble in polar solvents, so that it is necessary to add such products in the form of a salt with a base. Examples of the salts include salts of alkali metals such as sodium and potassium and ammonium salts, and salts of organic bases such as ethanolamine, diethanolamine, triethanolamine, 2-amino-2-methylpropanol, 2-(aminomethylthio)propane-1,3-diol, triisopropanolamine, glycine, histidine, and arginine. These salts may be prepared in a separately and added to the pre-shampoo treatment, or the product obtained by oxidation of keratin material or the chemically modified reduction derivative of keratin material and a base may be added to the pre-shampoo treatment composition in which they are converted into the desired salt. In the latter case, the bases are, for example, inorganic bases such as sodium hydroxide, potassium hydroxide, sodium carbonate and potassium carbonate and organic bases such as ammonia, ethanolamine, diethanolamine, triethanolamine, 2-amino-2-methyl-1-propanol, 2-amino-2-methyl-1,3-propanediol, 2-amino-2-methyl-1-propanol, 2-amino-2-ethyl-1,3-propanediol, 2-amino-1-butanol, triisopropanolamine, diisopropanolamine, monoisopropanolamine, lysine, arginine, histidine and hydroxylysine. Where the bases are added to the system to form a salt, the amount of the bases is preferably 0.1—8 equivalents of the carboxyl group or sulfonic acid group of the product obtained by the method (1) or (2).

The cationic polymers to be used in another modification of the present invention include polymeric materials of diallyl quaternary ammonium salts, cationic celluloses, cationic starches, and cationic vinyl polymers which are described in the following.

1 Polymeric materials of diallyl quaternary ammonium salts

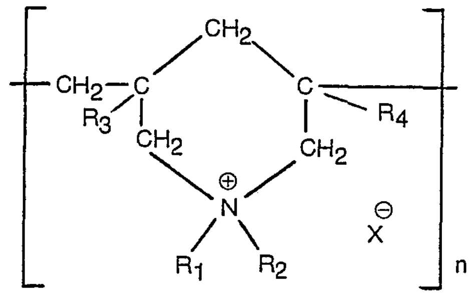

(1)

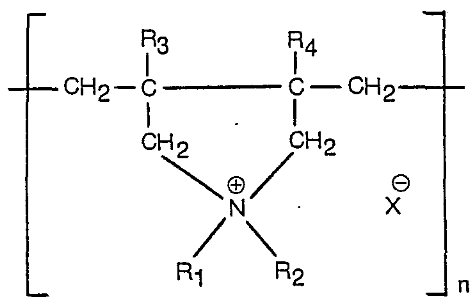

(2)

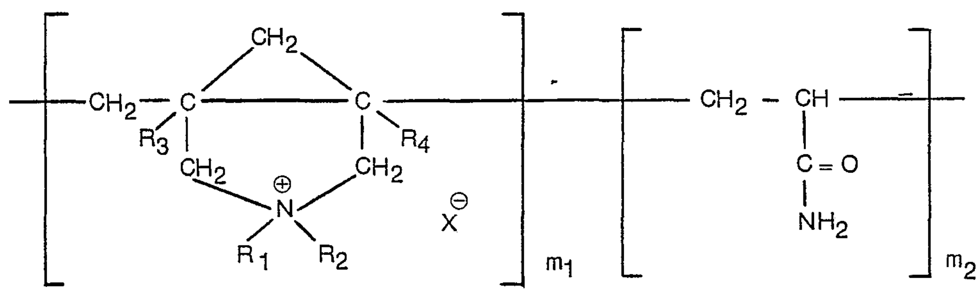

(3)

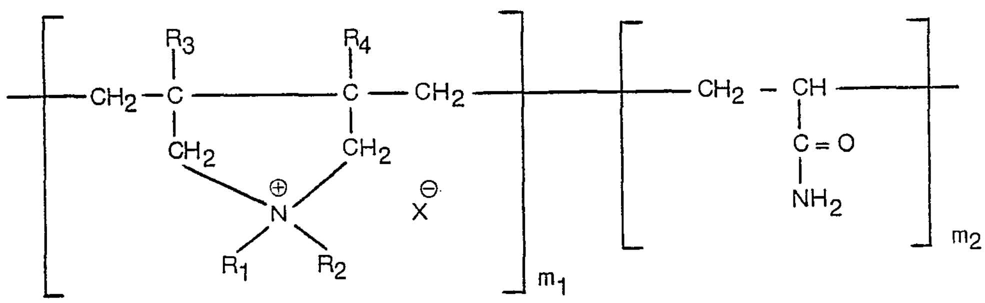

(4)

(in which $R_1$ and $R_2$ may be the same or different and represent hydrogen or an alkyl group having 1—18, preferably 1—4, carbon atoms, $R_3$ and $R_4$ may be the same or different and represent hydrogen, an alkyl group having 1—3 carbon atoms or a phenyl group, $X^{\ominus}$ represents an anion, e.g. a halide ion such as chlorine or bromine, an anion of an inorganic acid such as sulfuric acid or nitric acid, or an anion of an organic acid such as methylsulfuric acid, or a carboxylic acid, and n, $m_1$ and $m_2$ are values enough to give a molecular weight of 10,000—1,000,000).

2 Cationic celluloses or cationic starches

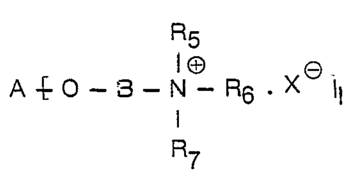

(5)

(in which

A: a residue of cellulose or starch.

B: an alkylene group or a hydroxyalkylene group,

$R_5$, $R_6$, $R_7$: They may be the same or different and represent an alkyl group, an aryl group, an aralkyl group or may form a hyterocyclic ring by combination with the nitrogen atom in the formula,

X: an anion (chlorine, bromine, iodine or an anion of sulfuric acid, sulfonic acid, methylsulfuric acid, phosphoric acid or nitric acid)

l: a positive integer.

3 Cationic vinyl polymers

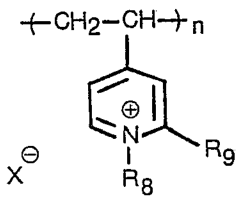

(6)

(in which $R_8$ and $R_9$ may be the same or different and represent hydrogen, an alkyl group having 1—6 carbon atoms or a phenyl group, and n and $X^{\ominus}$ have the same meanings as defined hereinbefore, respectively.

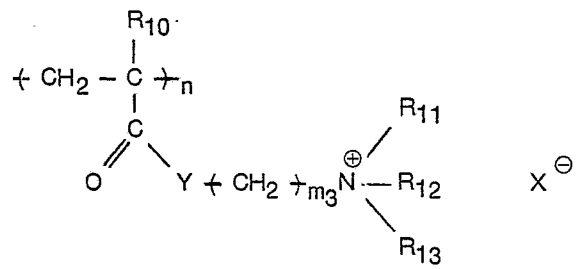

(7)

(in which

$R_{10}$: a hydrogen atom or a methyl group,

$R_{11}$, $R_{12}$, $R_{13}$: They may be the same or different and represent a hydrogen atom, an alkyl group having 1—4 carbon atoms, or a substituted alkyl group,

Y: an oxygen atom or an NH group,

X: an anion,

$m_3$: an integer of 1 to 10,

n: having the same meaning as defined hereinbefore.

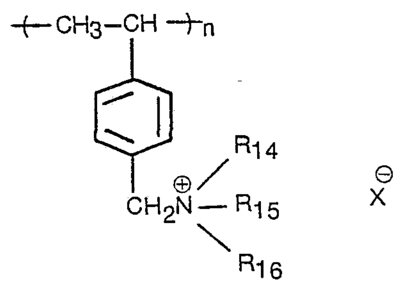

(8)

(in which

$R_{14}$, $R_{15}$, $R_{16}$: They are the same or different and represent a hydrogen atom, an alkyl group having 1 or 2 carbon atoms, or a substituted alkyl group.

X: an anion,

n: having the same meaning as defined hereinbefore.

Of these cationic polymers, diallyldimethylammonium homopolymer and cationic cellulose are preferred.

In the pre-shampoo treatment compositions according to the invention the amount of the keratin derivative is not critical but amounts less than 0.05 wt% (hereinafter referred to simply as %) encountered difficulty in showing satisfactory effects of the invention whereas amounts larger than 5% are disadvantageous in that stickiness develops under high humidity conditions. Accordingly, the amount is preferably in the range of 0.05—5% of the composition.

In the second embodiment according to the invention, the amount of the cationic polymer is preferably in the range of 0.1—5% of the composition since amounts less than 0.1% are unfavorable in the sense that the effect of the polymer is not shown whereas amounts larger than 5% do not offer any further advantages.

In the second embodiment of the invention, the ratio by weight of the keratin derivative to the cationic polymer is generally in the range of 1:10—20:1, preferably 1:5—10:1.

The pre-shampoo treatment composition according to one embodiment of the invention can be prepared by dissolving or suspending the keratin derivative and other known components optionally added as required in a medium such as water. Likewise, the pre-shampoo treatment composition according to another embodiment of the invention can be prepared by dissolving or suspending the keratin derivative, cationic polymer and other known components optionally added as required in a medium such as water.

The known optional components include oils such as higher alcohols and fatty acid esters, nonionic surface active agents serving as emulsifiers or solubilizing agent such as polyoxyalkylene alkyl ethers, moisture-holding agents such as glycerine and pyrrolidonecarboxylic acids. By using these additives, the finishing feeling of the pre-shampoo treatment after treatment and washing of hair can be arbitrarily controlled. That is, the addition of a liquid oil component contributes by imparting suppleness to the hair, the moisture-holding agents contribute by imparting the feeling of moistness to the hair, and higher alcohols serve to impart the feeling of dryness.

The thus obtained pre-shampoo treatment composition according to one embodiment of the invention comprises keratin derivatives which show a better absorptivity on the hair than other peptide compounds such as, for example, collagen, so that it shows good moisture retentivity accompanied by the effect of other polar groups, with the attendant advantages that hair can be finished supplely and that the pre-shampoo can impart good conditioning effects to hair without involving any dullness which is considered as an inherent disadvantage of known products. Furher the pre-shampoo treatment composition according to the second embodiment of the invention shows a better conditioning effect when adsorbed on the hair since cationic polymers with large moisture retentivity are contained together with the keratin derivatives and especially shows an unexpectedly excellent conditioning effect on hair which has suffered damages.

The present invention is particularly described by way of Synthetic Examples and Examples, which should not be construed as limiting the present invention thereto.

Synthetic Example 1

Preparation of oxidation, derivatives of keratin materials:

(a) Ten grams of wool fibers were immersed in 700 g of 8% aqueous peracetic acid solution at room temperature for 1 day. The resulting oxidized wool was filtered and washed with water, and then immersed in 700 g of a 0.1N aqueous ammonia at room temperature for 1 day, about 90% of the wool dissolved in the ammoniacal solution. About 1 g of the insoluble material was removed by filtration and the aqueous ammoniacal solution of keratose (oxidized product of wool keratin) was admixed with 2N hydrochloric acid to adjust the pH to 4.0 whereupon α-keratose precipitated. This precipitate was filtered, washed with acetone and dried to yield 5.4 g of α-keratose.

(b) Wool fibers were heated under pressure in an autoclave by the use of saturated steam at 6 $kg/cm^2$ for 6 minutes and were abruptly released into the air to obtain a porous swollen material. Ten grams of the swollen material which had been reduced to pieces, were immersed in 250 g of formic acid and 50 g of a 30% aqueous hydrogen peroxide solution in a 500 ml three neck flask. The mixture was allowed to stand at room temperature for 1 day, whereupon no powder was found in the solution but a foam-like material floated on the upper layer. This reaction mixture was filtered and the filtrate was poured into 1.5 liters of water, followed by adding hydrochloric acid to adjust the pH to 4. The resulting precipitate was collected by filtration and washed with 500 ml of water to obtain 4.5 g of α-keratose. The insoluble material from which the reaction product had been removed by filtration were immersed in 350 ml of water and then the pH of the mixture was adjusted to 11 by adding aqueous ammonia. The mixture was allowed to stand at room temperature for 1 day. The mixture was filtered and the pH of the filtrate was adjusted to 4 by adding hydrochloric acid. The resulting precipitate was collected by filtration to obtain 0.7 g of α-keratose. It was found that the 1.4 g of the insoluble material was primarily β-keratose.

Synthetic Example 2

Preparation of reduced derivatives of keratin materials:

(a) Ten grams of wool fibers were immersed in 600 ml of an aqueous solution of 8 M urea and 0.01 M Tris buffer, to which was added 6 ml of 2-mercaptoethanol, followed by adjusting the pH to 10 by means of a 5N aqueous potassium hydroxide. The reduction was carried out in a stream of nitrogen at room temperature. About 3 hours after commencement of the reaction, 85% of the wool dissolved in the reaction solution. While adjusting the pH of the reaction mixture with a 5N potassium hydroxide aqueous solution so as not to permit the pH below 7, 16.5 g of iodoacetic acid was gradually added and the pH of the system was finally adjusted to 8.5. The carboxymethylation reaction was carried out at room temperature for 2 hours. The reaction solution was filtered to remove insoluble material therefrom and the resultant filtrate was charged into a cellulose tube wherein it was dialyzed against deionized water to remove low molecular weight impurities including urea. As the urea was dialyzed, the contents in the cellulose tube turned white since HGT (component with high contents of glycine and tyrosine) water-insoluble component precipitated. After completion of the dialysis, the HGT was removed by centrifugal separation and S-carboxymethyl keratin (SCMKA) was obtained from the neutral transparent aqueous solution of SCMKA by

the isoelectric precipitation. That is, IN hydrochloric acid was added to the system to adjust its pH to 4.4 when the SCMKA precipitated. This precipitate was filtered, washed with ethanol and dried to obtain 4.2 g of SCMKA.

(b) The procedure of Synthetic Example 2-(a) was repeated except that feathers were used instead of wool fibers. The feathers were heated for 6 minutes in an autoclave by means of superheated steam at 6 kg/$cm^2$ and 240°C and then abruptly released into the air to obtain a porous swollen material and that 1.75 g of maleic acid was used instead of iodoacetic acid, thereby obtaining 5.3 g of S-(1,2-dicarboxyethyl)-keratin.

(c) The procedure of Synthetic Example 2-(a) was repeated using powdered horses' hooves instead of wool fibers and 11 g of acrylic acid instead of iodoacetic acid, thereby obtaining 4.2 g of S-(2-carboxyethyl)keratin.

(d) The procedure of Synthetic Example 2-(a) was repeated using 28 g of p-styrenesulfonic acid instead of iodoacetic acid, thereby obtaining 4.8 g of S-(p-sulfophenylvinyl)-keratin.

(e) Eight grams of wool fibers were dispersed in 300 ml of n-propanol and 300 ml of 0.1N Tris buffer solution. After displacing the air with nitrogen, 3.2 ml of tri-n-butylphosphine was added, followed by agitating at room temperature for 24 hours. After filtration, 400 ml of water, 9.28 g of maleic acid and about 30 ml of 5N potassium hydroxide to adjust the pH to 8.0 were added to the insoluble material, followed by agitating at room temperature for 6 hours. To the reaction mixture was added about 20 ml of a 28% aqueous ammonia to adjust the pH to 11.5, after which it was agitated at room temperature for 18 hours. The reaction solution was filtered to remove impurities therefrom and the resultant filtrate was placed in a cellulose tube in which it was dialyzed against ion-exchanged water to remove low molecular weight impurities therefrom. After completion of the dialysis, the insoluble material in the cellulose tube were removed by centrifugal separation and the neutral transparent aqueous solution was adjusted to a pH of 4.4 by addition of about 5.5 ml. of 1N hydrochloric acid and the resulting precipitate was collected by filtration, followed by washing with ethanol and drying to obtain 3.9 g of S-(1,2-dicarboxyethyl)-keratin.

(f) The procedure of Synthetic Example 2-(e) was repeated except that a powder of a porous swollen material which was obtained by heating wool in an autoclave by means of saturated steam at 6 kg/$cm^2$ for 6 minutes were used instead of wool fibres and that 16.5 g of 2-acrylamido-2-methylpropane-sulfonic acid was used instead of maleic acid, thereby obtaining 4.5 g of S-[2-(1,1-dimethyl-2-sulfoethylcarbamoyl)-ethyl]-keratin.

## Example 1

Pre-shampoo treatment composition of the following formulations were prepared using derivatives of keratin materials to evaluate their performance. The results are shown in Table 2.

Formulation:

| | |
|---|---|
| Derivatives of keratin materials (those shown in Table 1) | (Table 1) |
| Lauryl alcohol | 2.0% |
| Polyoxyethylene (15) nonyl phenyl ether | 0.5 |
| Hydroxyethyl cellulose | 0.5 |
| Ethanol | 5.0 |
| Triethanolamine | (Table 1) |

Evaluation method:

(1) Feel of the hair during washing

Tresses of hair of Japanese females with a length of 20 cm and a weight of 20 g were each treated with 2 g of each of pre-shampoo treatment compositions. After being allowed to stand for 5 minutes, the hair was treated with 2 g of a commercially available plain shampoo and lathered as usual for 1 minute, whereupon the feeling of the hair was evaluated. The evaluation was made by a paired comparison test in which a tress treated with a commercially available pre-shampoo hair treatment mainly composed of lanolin was used as a control.

(The evaluation was indicated by an average value of an expert panel consisting of 20 members).

| Evaluation Point | Evaluation |
|---|---|
| +2 | Better feel than that of the control hair tress. |
| +1 | Slightly better feel than that of the control hair tress. |
| 0 | Feel equal to that of the control hair tress. |
| −1 | Slightly poorer feel than that of the control hair tress. |
| −2 | Poorer feel than that of the control hair tress. |

(2) Feel of the wet Hair

Ater completion of the evaluation of the feel of the hair during washing, it was washed with running water of 40°C for 1 minute and was dried with a towel to remove excess water. The tress was evaluated similarly to the tress during washing to evaluate the feel of the wet hair.

(3) Feel and combing ease after drying

After the evaluation of (2), the wet hair tresses were each air dried and its feel was evaluated in accordance with the method of (2). Then, the combing ease was evaluated similarly to the method of (1) using a commercially available nylon comb.

TABLE 1

| Sample No. | Derivative of keratin material | Amount | Amount of triethanolamine |
|---|---|---|---|
| 1 | derivative of keratin material of Synthetic Example 1–(a) | 0.1% | 0.01% |
| 2 | ,, | 0.5 | 0.05 |
| 3 | ,, | 2.0 | 0.1 |
| 4 | ,, | 5.0 | 0.5 |
| 5 | derivative of keratin material of Synthetic Example 1–(b) | 2.0 | 0.2 |
| 6 | derivative of keratin material of Synthetic Example 2–(a) | 2.0 | 0.2 |
| 7 | derivative of keratin material of Synthetic Example 2–(b) | 0.1 | 0.01 |
| 8 | ,, | 0.5 | 0.05 |
| 9 | ,, | 2.0 | 0.2 |
| 10 | ,, | 5.0 | 0.5 |
| 11 | derivative of keratin material of Synthetic Example 2–(c) | 2.0 | 0.2 |
| 12 | derivative of keratin material of Synthetic Example 2–(d) | 2.0 | 0.2 |
| 13 | derivative of keratin material of Synthetic Example 2–(e) | 2.0 | 0.2 |
| 14 | derivative of keratin material of Synthetic Example 2–(f) | 2.0 | 0.2 |
| Comparative Product 1 | derivative obtained by decomposition of collagen with acid (M.W. 10,000–20,000) | 2.0 | 0 |
| Comparative Product 2 | derivative obtained by decomposition of collagen with alkali (M.W. 800–1,000) | 3.0 | 0 |
| Control 1 | nil | 0 | 0 |

Results:

TABLE 2

| Sample No. | Feel of Hair during washing | Feel of Hair after washing | Feel of Hair after drying | Combing Ease (after drying) |
|---|---|---|---|---|
| 1 | +0.9 | +1.0 | +0.9 | +0.8 |
| 2 | +1.1 | +1.2 | +1.2 | +1.1 |
| 3 | +1.3 | +1.4 | +1.4 | +1.3 |
| 4 | +1.4 | +1.4 | +1.4 | +1.4 |
| 5 | +1.3 | +1.3 | +1.3 | +1.4 |
| 6 | +1.3 | +1.4 | +1.3 | +1.3 |
| 7 | +0.9 | +0.9 | +1.0 | +0.9 |
| 8 | +1.1 | +1.1 | +1.2 | +1.2 |
| 9 | +1.3 | +1.4 | +1.3 | +1.4 |
| 10 | +1.4 | +1.4 | +1.4 | +1.3 |
| 11 | +1.0 | +1.1 | +1.1 | +1.0 |
| 12 | +1.3 | +1.4 | +1.4 | +1.4 |
| 13 | +1.3 | +1.4 | +1.3 | +1.4 |
| 14 | +1.4 | +1.4 | +1.3 | +1.4 |
| Comparative Product 1 | +0.4 | +0.5 | +0.4 | +0.4 |
| Comparative Product 2 | +0.3 | +0.4 | +0.4 | +0.3 |
| Control 1 | 0 | 0 | 0 | 0 |

Example 2

Pre-shampoo treatment compositions having the following formulations were prepared using derivatives of keratin materials and polymers having cyclic cationic groups therein and their performance was evaluated in accordance with the method of Example 1. The results were shown in Table 4.

Formulation:

| | |
|---|---|
| Poly(dimethyldiallylammonium chloride) (M.W. 100,000) | 1.0% |
| Derivatives of keratin materials (Table 3) | (Table 3) |
| Polyoxyethylene (10) oleyl ether | 2.0 |
| Hydroxyethyl cellulose | 0.5 |
| Propylene glycol | 5.0 |
| Triethanolamine | (Table 3) |

TABLE 3

| Sample No. | Derivative of keratin material | Amount | Amount of triethanolamine |
|---|---|---|---|
| 1 | derivative of keratin material of Synthetic Example 1—(a) | 0.1% | 0.01% |
| 2 | ,, | 0.5 | 0.05 |
| 3 | ,, | 1.0 | 0.1 |
| 4 | ,, | 2.0 | 0.2 |
| 5 | ,, | 5.0 | 0.5 |
| 6 | derivative of keratin material of Synthetic Example 1—(b) | 2.0 | 1.0 |
| 7 | derivative of keratin material of Synthetic Example 2—(a) | 2.0 | 0.3 |
| 8 | derivative of keratin material of Synthetic Example 2—(b) | 2.0 | 0.3 |
| 9 | derivative of keratin material of Synthetic Example 2—(c) | 2.0 | 0.3 |
| 10 | derivative of keratin material of Synthetic Example 2—(d) | 2.0 | 0.3 |
| 11 | derivative of keratin material of Synthetic Example 2—(e) | 2.0 | 0.3 |
| 12 | derivative of keratin material of Synthetic Example 2—(f) | 2.0 | 0.3 |
| Control 1 | Nil | 0 | 0 |

Results:

TABLE 4

| Sample No. | Feel of Hair | | | Combing Ease (after drying) |
|---|---|---|---|---|
| | during washing | after washing in wet state | after drying | |
| 1 | +1.1 | +1.2 | +1.3 | +1.2 |
| 2 | +1.4 | +1.5 | +1.5 | +1.4 |
| 3 | +1.6 | +1.6 | +1.5 | +1.5 |
| 4 | +1.7 | +1.7 | +1.6 | +1.6 |
| 5 | +1.8 | +1.8 | +1.7 | +1.6 |
| 6 | +1.8 | +1.7 | +1.7 | +1.7 |
| 7 | +1.7 | +1.7 | +1.8 | +1.7 |
| 8 | +1.8 | +1.8 | +1.8 | +1.8 |
| 9 | +1.4 | +1.5 | +1.5 | +1.3 |
| 10 | +1.7 | +1.7 | +1.7 | +1.7 |
| 11 | +1.8 | +1.7 | +1.8 | +1.6 |
| 12 | +1.7 | +1.7 | +1.8 | +1.6 |
| Control 1 | +0.6 | +0.7 | +0.7 | +0.6 |
| No treatment | −0.3 | 0 | 0 | 0 |

Example 3.

Pre-shampoo treatment compositions of the following formulations were prepared using the derivatives of keratin materials prepared in Synthetic Examples 3-(b) and the cationic polymers shown in Table 5 and their performance was evaluated in accordance with the method of Example 1. The results were shown in Table 6.

Formulation:

| | |
|---|---|
| Derivative of keratin materials (Synthetic Example 3-(b) | 2.0% |
| Cationic polymers (Table 5) | (Table 5) |
| Polyoxyethylene (20) nonyl phenyl ether | 2.0 |
| Methyl cellulose | 0.3 |
| Propylene glycol | 5.0 |

TABLE 5

| Sample No. | Cationic Polymer | Amount (%) |
|---|---|---|
| 1 | 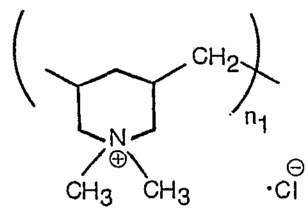 (average M.W. 100,000) | 0.5 |
| 2 | ,, | 1.0 |
| 3 | ,, | 2.0 |
| 4 | 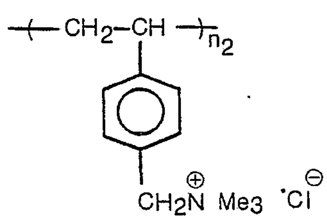 (average M.W. 20,000) | 1.0 |
| 5 | 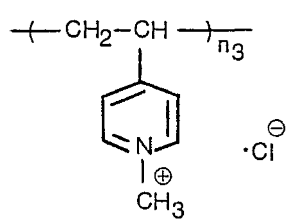 (average M.W. 20,000) | 1.0 |
| 6 | cationic cellulose (Polymer JR–400, made by UCC Co., Ltd.) | 1.0 |
| 7 | ,, | 2.0 |

TABLE 5 (Continued)

| Sample No. | Cationic Polymer | Amount (%) |
|---|---|---|
| 8 | 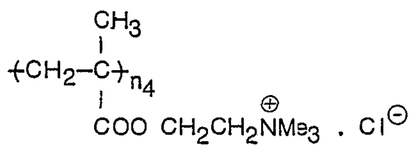 (average M.W. 1,000,000) | 0.5 |
| 9 | ,, | 1.0 |
| 10 | ,, | 2.0 |
| 11 | cationic product of vinylpyrrolidonedimethylaminoethyl-methacrylate copolymer (GAF Coat 734; made by GAF. Co., Ltd.) | 1.0 |
| 12 | aminoethyl acrylate-methacrylate acid-methacrylic ester copolymer (Cartolex L: National Starch Co., Ltd.) | 1.0 |
| 13 | 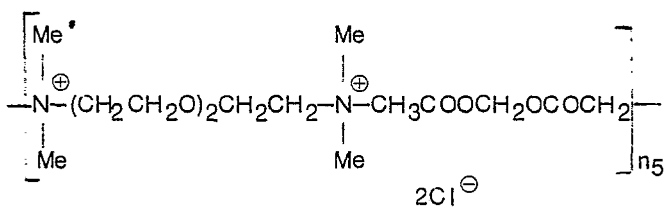 (average M.W. 100,000) | 1.0 |
| 14 | cationic starch (the average number of cationic groups introduced per anhydrous glucose unit is 0.30 and the viscosity of its 1% aqueous solution at 50°C is 30 centipoises) | 1.0 |
| 15 | nil | 0 |

Results:

TABLE 6

| Sample No. | Feel of Hair during washing | Feel of Hair after washing in wet state | Feel of Hair after drying | Combing Ease (after drying) |
|---|---|---|---|---|
| 1 | +1.7 | +1.7 | +1.7 | +1.7 |
| 2 | +1.7 | +1.8 | +1.7 | +1.8 |
| 3 | +1.8 | +1.8 | +1.8 | +1.8 |
| 4 | +1.6 | +1.6 | +1.6 | +1.5 |
| 5 | +1.6 | +1.5 | +1.6 | +1.6 |
| 6 | +1.8 | +1.8 | +1.8 | +1.8 |
| 7 | +1.8 | +1.8 | +1.8 | +1.8 |
| 8 | +1.5 | +1.5 | +1.4 | +1.4 |
| 9 | +1.6 | +1.6 | +1.5 | +1.4 |
| 10 | +1.7 | +1.6 | +1.6 | +1.5 |
| 11 | +1.5 | +1.5 | +1.5 | +1.4 |
| 12 | +1.5 | +1.5 | +1.5 | +1.5 |
| 13 | +1.6 | +1.7 | +1.6 | +1.7 |
| 14 | +1.4 | +1.5 | +1.4 | +1.3 |
| 15 | +0.4 | +0.7 | +0.8 | +0.4 |
| No treatment | −0.3 | 0 | 0 | 0 |

**Claims for the Contracting States: BE CH DE FR IT LI NL**

1. A pre-shampoo hair treatment composition which comprises a suspension or solution, in a polar solvent, of at least one keratin derivative, selected from 1) salts with bases of products obtained by oxidation of keratin materials, and 2) salts with bases of derivatives obtained by chemically modifying the mercapto group of a product obtained by reduction of keratin material, wherein said derivatives are selected from

$-SCH_2COOH$, $-SCH_2CH_2COOH$, 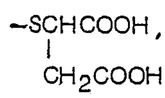 ,

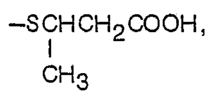 , 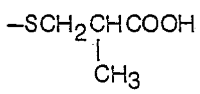 ,

$-SSO_3H$, $-SCH_2CH_2SO_3H$, 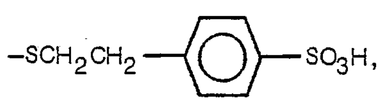 ,

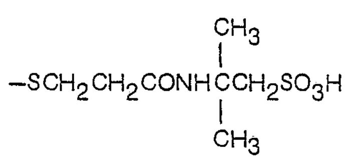 , and $SCH_2CH_2SO_3CH_2COOH$.

2. A pre-shampoo hair treatment composition according to Claim 1, wherein said derivative is contained in an amount of 0.05 to 5 wt% of the composition.

3. A pre-shampoo hair treatment composition according to Claim 1 or Claim 2, further comprising at least one cationic polymer in an amount of 0.1 to 5 wt % of the composition.

4. A pre-shampoo hair treatment composition according to Claim 3, wherein said cationic polymer is a member selected from diallyl quaternary ammonium salts, cationic celluloses, cationic starches and cationic vinyl polymers.

5. A pre-shampoo hair treatment composition according to Claim 3 or 4, wherein said cationic polymer is diallyldimethylammonium homopolymer.

6. A pre-shampoo hair treatment composition according to Claim 3 or 4, wherein said cationic polymer is cationic cellulose.

7. A pre-shampoo hair treatment composition according to Claim 3, wherein the ratio by weight of the said keratin derivative to the cationic polymer is in the range of 1:10 to 20:1.

8. A pre-shampoo hair treatment composition according to Claim 7, wherein the ratio is in the range of 1:5 to 10:1.

**Claims for the Contracting State: AT**

1. A method for producing a pre-shampoo hair treatment composition which comprises suspending or dissolving, in a polar solvent, at least one keratin derivative selected from 1) salts with bases of products obtained by oxidation of keratin material, and 2) salts with bases of derivatives obtained by chemically modifying the mercapto group of a product obtained by reduction of keratin material, wherein said derivatives are selected from

$-SCH_2COOH$, $-SCH_2CH_2COOH$, 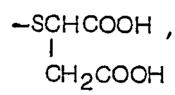 ,

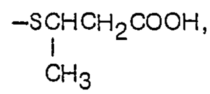 , 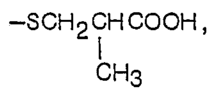 ,

$-SSO_3H$, $-SCH_2CH_2SO_3H$, 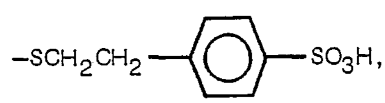 ,

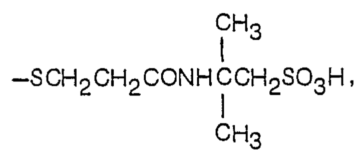 , and $SCH_2CH_2SO_3CH_2COOH$.

2. A method according to Claim 1, wherein said keratin derivative is admixed so as to be present in an amount of 0.05 to 5 wt% of the composition.

3. A method according to Claim 1 or Claim 2, further comprising admixing at least one cationic polymer in an amount of 0.1 to 5 wt% of the composition.

4. A method according to Claim 3, wherein said cationic polymer is selected from diallyl quaternary ammonium salts, cationic celluloses, cationic starches and cationic vinyl polymers.

5. A method according to Claim 3 or 4, wherein said cationic polymer is diallyldimethylammonium homopolymer.

6. A method according to Claim 3 or 4, wherein said cationic polymer is cationic cellulose.

7 . A method according to Claim 6, wherein the ratio by weight of the said keratin derivative to the cationic polymer is in the range of 1:10 to 20:1.

8. A method according to Claim 7, wherein the ratio is in the range of 1:5 to 10:1.

9. A method for the treatment of hair which comprises applying thereto a product of any one of Claims 1 to 8.

**Patentansprüche für die Vertragsstaaten: BE CH DE FR IT LI NL**

1. Vorshampoo-Haarbehandlungsmittel, umfassend eine Suspension oder Lösung in einem polaren Lösungsmittel von mindestens einem Keratinderivat, ausgewählt aus

1) Basensalzen von Produkten, die erhalten wurden durch die Oxidation von Keratinmaterialien und

2) Basensalzen von Derivaten, die erhalten wurden durch chemische Modifikation der Merkaptogruppe eines Produkts, daß durch Reduktion von Keratinmaterial erhalten wurde, wobei die Derivate ausgewählt sind aus

$-SCH_2COOH$, $-SCH_2CH_2COOH$, 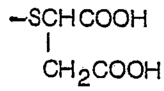

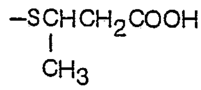, 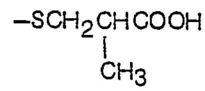,

$-SSO_3H$, $-SCH_2CH_2SO_3H$, 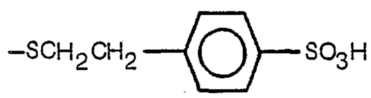

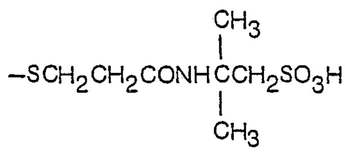, und $SCH_2CH_2SO_3CH_2COOH$.

2. Vorshampoo-Haarbehandlungsmittel nach Anspruch 1, wobei das Derivat in einer Menge von 0,05 bis 5 Gew.% des Mittels enthalten ist.

3. Vorshampoo-Haarbehandlungsmittel nach Anspruch 1 oder Anspruch 2, welches außerdem mindestens ein kationisches Polymeres in einer Menge von 0,1 bis 5 Gew.% des Mittels umfaßt.

4. Vorshampoo-Haarbehandlungsmittel nach Anspruch 3, wobei das kationische Polymere ausgewählt ist aus der Gruppe Diallylquaternäre Ammoniumsalze, kationische Zellulosen, kationische Stärken und kationische Vinylpolymere.

5. Vorshampoo-Haarbehandlungsmittel nach Anspruch 3 oder 4, wobei das kationische Polymere Diallyldimethylammoniumhomopolymerisat ist.

6. Vorshampoo-Haarbehandlungsmittel nach Anspruch 3 oder 4, wobei das kationische Polymere kationische Zellulose ist.

7. Vorshampoo-Haarbehandlungsmittel nach Anspruch 3, wobei das Gewichtsverhältnis des Keratinderivates zu dem kationischen Polymeren im Bereich von 1:10 bis 20:1 liegt.

8. Vorshampoo-Haarbehandlungsmittel nach Anspruch 7, wobei das Verhältnis im Bereich von 1:5 bis 10:1 liegt.

**Patentansprüche für den Vertragsstaat: AT**

1. Verfahren zur Herstellung eines Vorshampoo-Haarbehandlungsmittels, wobei man mindestens ein Keratinderivat, ausgewählt aus

1) Basensalzen von Produkten, die durch Oxidation von Keratinmaterial erhalten wurden, und

2) Basensalzen von Derivaten, die erhalten wurden durch chemische Modifikation der Mercaptogruppe eines Produktes, das durch Reduktion von Keratinmaterial erhalten wurde, wobei die Derivate ausgewählt sind aus

$-SCH_2COOH$, $-SCH_2CH_2COOH$, 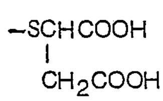

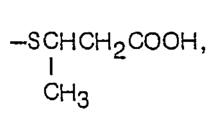, 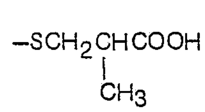,

$-SSO_3H$, $-SCH_2CH_2SO_3H$, 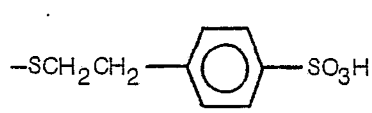

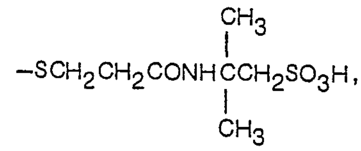 und $SCH_2CH_2SO_3CH_2COOH$,

in einem polaren Lösungsmittel suspendiert oder auflöst.

2. Verfahren nach Anspruch 1, wobei das Keratinderivat in der Weise zugemischt wird, daß es in einer Menge von 0,05 bis 5 Gew.% des Mittels vorliegt.

3. Verfahren nach Anspruch 1 oder Anspruch 2, umfassend ferner das Zumischen von mindestens einem kationischen Polymeren in einer Menge von 0,1 bis 5 Gew.% des Mittels.

4. Verfahren nach Anspruch 3, wobei das kationische Polymere ausgewählt ist aus Diallyl-quaternären Ammoniumsalzen, kationischen Zellulosen, kationischen Stärken und kationischen Vinylpolymeren.

5. Verfahren nach Anspruch 3 oder 4, wobei das kationische Polymere Diallyldimethylammoniumhomopolymerisat ist.

6. Verfahren nach Anspruch 3 oder 4, wobei das kationisches Polymere kationische Zellulose ist.

7. Verfahren nach Anspruch 6, wobei das Gewichtsverhältnis des Keratinderivates zu dem kationischen Polymeren im Bereich von 1:10 bis 20:1 liegt.

8. Verfahren nach Anspruch 7, wobei das Verhältnis im Bereich von 1:5 bis 10:1 liegt.

9. Verfahren zur Haarbehandlung, umfassend die Applikation eines Produktes gemäß der Ansprüche 1 bis 8.

**Revendications pour Les Etats contractants: BE CH DE FR IT LI NL**

1. Composition pour le traitement des cheveux du type pré-shampooing, qui comprend une suspension ou solution, dans un solvant polaire, d'au moins un dérivé de kératine choisi parmi (1) les sels avec des bases de produits obtenus par l'oxydation de matériaux kératiniques et (2) les sels avec des bases de dérivés obtenus par modification chimique du groupe mercapto d'un produit obtenu par réduction d'un matériau kératinique, lesdits dérivés étant choisis parmi

$-SCH_2COOH$, $-SCH_2CH_2COOH$, 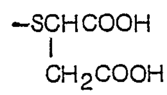

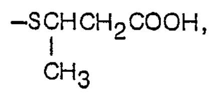, 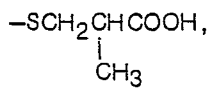,

$-SSO_3H$, $-SCH_2CH_2SO_3H$, 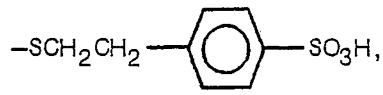,

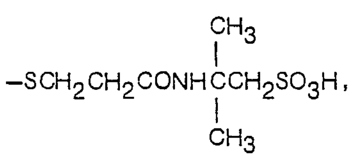 et $SCH_2CH_2SO_3CH_2COOH$.

2. Composition pour le traitement des cheveux du type pré-shampooing selon la revendication 1, dans laquelle ledit dérivé est présent à raison de 0,05 à 5% en pds de la composition.

3. Composition pour le traitement des cheveux du type pré-shampooing selon la revendication 1 ou 2, qui comprend en outre au moins un polymère cationique à raison de 0,1 á 5% en pds de la composition.

4. Composition pour le traitement des cheveux du type pré-shampooing selon la revendication 3, dans laquelle ledit polymère cationique est un élément choisi dans le groupe comprenant les sels de diallyl ammonium quaternaire, les celluloses cationiques, les amidons cationiques et les polymères vinyliques cationiques.

5. Composition pour le traitement des cheveux du type pré-shampooing selon la revendication 3 ou 4, dans laquelle ledit polymère cationique est un homopolymère de diallyldiméthyl ammonium.

6. Composition pour le traitement des cheveux du type pré-shampooing selon la revendication 3 ou 4, dans laquelle ledit polymère cationique est une cellulose cationique.

7. Composition pour le traitement des cheveux du type pré-shampooing selon la revendication 3, dans laquelle le rapport en poids dudit dérivé de kératine au polymère cationique est situé dans la plage allant de 1:10 à 20:1.

8. Composition pour le traitement des cheveux du type pré-shampooing selon la revendication 7, dans laquelle le rapport est situé dans la plage allant de 1:5 à 10:1.

**Revendications pour l'Etat contractant: AT**

1. Procédé pour produire une composition pour le traitement des cheveux du type pré-shampooing, qui consiste à mettre en suspension ou à dissoudre, dans un solvant polaire, au moins un dérivé de kératine choisi parmi (1) les sels avec une base de produits obtenus par oxydation d'un matériau kératinique et (2) les sels avec des bases de dérivés obtenus par modification chimique du groupe mercapto d'un produit obtenu par réduction d'un matériau kératinique, lesdits dérivés étant choisis parmi

$-SCH_2COOH$, $-SCH_2CH_2COOH$, 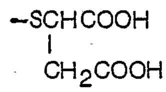

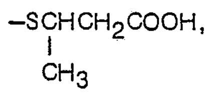, 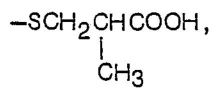,

$-SSO_3H$, $-SCH_2CH_2SO_3H$, 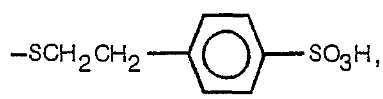,

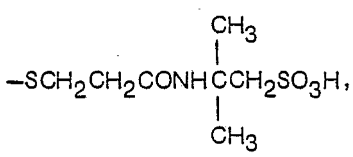, et $SCH_2CH_2SO_3CH_2COOH$.

2. Procédé selon la revendication 1, dans lequel on mélange ledit dérivé de kératine de manière qu'il soit présent à raison de 0,05 à 5% en pds de la composition.

3. Procédé selon la revendication 1 ou 2, comportant en outre l'adjonction par mélange d'au moins un polymère cationique à raison de 0,1 à 5% en pds de la composition.

4. Procédé selon la revendication 3, dans lequel ledit polymère cationique est choisi parmi les sels de diallyl ammonium quaternaire, les celluloses cationiques, les amidons cationiques et les polymères vinyliques cationiques.

5. Procédé selon la revendication 3 ou 4, dans lequel ledit polymère cationique est un homopolymère de diallyldiméthyl ammonium.

6. Procédé selon la revendication 3 ou 4, dans lequel ledit polymère cationique est une cellulose cationique.

7. Procédé selon la revendication 6, dans lequel le rapport en poids dudit dérivé de kératin au polymère cationique est situé dans la plage allant de 1:10 à 20:1.

8. Procédé selon la revendication 7, dans le rapport est situé dans la plage allant de 1:5 à 10:1.

9. Procédé de traitement des cheveux qui consiste à appliquer sur ceux-ci un produit selon l'une quelconque des revendications 1 à 8.